# FASCICULE DE BREVET EUROPEEN

(11) **EP 0 995 487 B1**
(45) Date de publication et mention de la délivrance du brevet: **11.08.2004**
(21) Numéro de dépôt: 99402605.2
(22) Date de dépôt: 21.10.1999
(51) Int. Cl.: B01F 17/00

(54) **Nouvelles compositions à base d'alkylpolyglycosides et d'alcools gras, notamment utiles pour la préparation d'émulsions fluides stables**
Insbesondere für die Herstellung von stabilen flüssigen Emulsionen verwendbare neue Zusammensetzungen aus Alkylpolyglykosiden und Fettalkoholen
New compositions based on alkylpolyglycosides and fatty alcohols, in particular for the preparation of stable fluid emulsions

(30) Priorité: 22.10.1998 FR 9813255
(43) Date de publication de la demande: 26.04.2000
(73) Titulaire: SOCIETE D'EXPLOITATION DE PRODUITS POUR LES INDUSTRIES CHIMIQUES, S.E.P.P.I.C., 75321 Paris Cédex 07 (FR)
(72) Inventeur: Amalric, Chantal, 81700 Blan (FR); Tabacchi, Guy, 81100 Castres (FR); Boiteux, Jean-Pierre, 81100 Castres (FR); Michel, Nelly, 94700 Maisons-Alfort (FR); Milius, Alain, 06000 Nice (FR)
(74) Mandataire: Hubert, Philippe

(56) Documents cités:
- FR-A- 2 734 496

## Description

La présente invention concerne une nouvelle famille de compositions à base d'alkylpolyglycosides et d'alcools gras, notamment utiles pour la préparation d'émulsions fluides stables.

L'invention trouve notamment application dans le domaine cosmétique.

Les alkylglycosides ou alkylpolyglycosides (APG) sont des composés tensioactifs non ioniques bien connus qui peuvent être utilisés seuls, ou en association avec d'autres tensioactifs, dans une large gamme d'applications industrielles et notamment dans le domaine cosmétique.

Les alkylpolyglycosides ont d'abord été utilisés comme agents moussants et dans cette application, ceux dont la chaîne alkyle comporte de 8 à 14 atomes de carbone se sont avérés particulièrement intéressants.

Plus récemment, les alkylpolyglycosides ont été utilisés comme émulsionnants, et dans cette application, ceux dont la chaîne alkyle comporte de 16 à 18 atomes de carbone se sont avérés particulièrement intéressants.

La demande de brevet WO 92/06778, au nom de la demanderesse, décrit pour la première fois l'utilisation de mélanges d'alkylpolyglycosides et d'alcools gras en tant qu'agents auto-émulsionnants.

Par "auto-émulsionnant", on désigne tout agent ou composition capable de former une émulsion stable avec une phase aqueuse, pratiquement sans apport d'énergie, par exemple par dispersion dans la phase aqueuse par agitation mécanique lente.

Plus précisément, les mélanges décrits dans ce document antérieur comprennent :
- de 60 à 90 % en poids d'au moins un alcool gras ayant de 12 à 22 atomes de carbone, et de préférence de 16 à 18 atomes de carbone ; et
- de 10 à 40 % en poids d'un alkylpolyglycoside, dont la partie alkyle est de préférence identique à celle de l'alcool gras.

Les compositions auto-émulsionnables décrites dans la demande précitée sont commercialisées sous la dénomination Montanov® 68 et comportent un mélange d'alkylpolyglycosides dont les chaînes grasses comprennent 16 et 18 atomes de carbone, ainsi qu'un mélange d'alcools gras de même longueur de chaînes grasses.

Si de telles compositions sont parfaitement satisfaisantes notamment au niveau de la stabilité des émulsions qu'elles permettent d'obtenir, elles ne permettent cependant pas d'obtenir aisément des émulsions fluides.

Par ailleurs, la demande de brevet internationale WO 95/13863 au nom de la demanderesse, décrit également des compositions à base d'alkylpolyglycosides et d'alcools gras, se présentant sous forme de concentrés, permettant la préparation d'émulsions fluides.

Ces compositions sont essentiellement caractérisées par le fait qu'elles comprennent un mélange d'au moins deux alkylpolyglycosides se différentiant par la nature de leur partie alkyle, l'un au moins de ces alkylpolyglycosides comportant une chaîne alkyle ayant de 16 à 22 atomes de carbone, et de préférence de 16 à 18 atomes de carbone et représentant au moins 25 %, et de préférence au moins 50 % en poids, du mélange d'alkylpolyglycosides.

Si les compositions décrites dans ce document antérieur sont satisfaisantes au niveau de la fluidité des émulsions qu'elles permettent d'obtenir, il a été observé que ces émulsions ne sont pas entièrement satisfaisantes du point de vue de la stabilité.

En pratique, il est généralement nécessaire de mettre en oeuvre de telles compositions à une dose d'utilisation élevée ou en association avec un co-tensioactif ou avec un stabilisant.

Dans ces conditions, la présente invention a pour but de résoudre le problème technique consistant en la fourniture de nouvelles compositions permettant à faible dose la préparation d'émulsions fluides stables, sans utilisation de co-tensioactif ou de stabilisant.

Il a été découvert, et ceci constitue le fondement de la présente invention, que certains mélanges particuliers d'alkylpolyglycosides et d'alcools gras permettent de répondre à l'objectif recherché.

Ainsi, la solution conforme à la présente invention pour résoudre le problème technique précité consiste en de nouvelles compositions à base d'alkylpolyglycosides et d'alcools gras, caractérisées en ce qu'elles comprennent :
* 5 à 60 % en poids d'un mélange d'alkylpolyglycosides essentiellement constitué de :
   - 10 à 25 % en poids d'un alkylpolyglycoside de formule (I) :

      R₁O(G₁)_{x₁} (I)
   - 10 à 30 % en poids d'un alkylpolyglycoside de formule (II) :

      R₂O(G₂)_{x₂} (II)
   - 0 à 10 % en poids d'un mélange d'alkylpolyglycosides de formules (III) et (IV):

      R₃O(G₃)_{x₃} (III)

      R₄O(G₄)_{x₄} (IV)
   - 40 à 80 % en poids d'un mélange d'alkylpolyglycosides de formules (V) et (VI) :

      R₅O(G₅)_{x₅} (V)

      R₆O(G₆)_{x₆} (VI)

      dans lesquelles R₁, R₂, R₃, R₄, R₅ et R₆ représentent chacun un radical aliphatique, linéaire ou ramifié, ayant respectivement 12, 14, 16, 18, 20 et 22 atomes de carbone, G₁, G₂, G₃, G₄, G₅ et G₆ représentent chacun le reste d'un saccharide, x₁, x₂, x₃, x₄, x₅ et x₆ représentent chacun un nombre compris entre 1 et 5 ;
* 95 à 40 % en poids d'un ou plusieurs alcools de formule R'OH, dans laquelle R' est un radical aliphatique, linéaire ou ramifié, ayant de **12** à 22 atomes de carbone, et de préférence d'un mélange constitué des alcools dont la partie alkyle est identique à la partie alkyle R₁, R₂, R₃, R₄, R₅ et R₆ des alkylpolyglycosides précités.

Les nouvelles compositions à base d'alkylpolyglycosides et d'alcools gras qui viennent d'être définies permettent d'une façon tout à fait inattendue la préparation d'émulsions fluides stables sans utilisation de co-tensioactif ou de stabilisant, et ce même à des doses d'utilisation relativement faibles.

Dans le cadre de la présente demande, on entend par "émulsion fluide", une émulsion dont l'écoulement au travers d'une coupe d'écoulement ISO 2431 de 6 mn commence moins de 5 s après l'enlèvement de l'obturateur (test selon la norme internationale ISO 243o1).

A titre d'émulsions fluides, on peut notamment citer les laits, en particulier des laits de type huile-dans-eau, à usage cosmétique ou hygiénique comme, par exemple, les laits démaquillants, les laits corporels ou les laits solaires.

Une sous-famille préférée de compositions à base d'alkylpolyglycosides et d'alcools gras susceptibles d'être utilisées dans le cadre de la présente invention est constituée des compositions dont le mélange d'alkylpolyglycosides est essentiellement constitué de :
- 10 à 20 % en poids d'un alkylpolyglycoside de formule (I) :

   R₁O(G₁)_{x₁} (I)
- 15 à 25 % en poids d'un alkylpolyglycoside de formule (II) :

   R₂O(G₂)_{x₂} (II)
- 0 à 10 % en poids d'un mélange d'alkylpolyglycosides de formules (III) et (IV):

   R₃O(G₃)_{x₃} (III)

   R₄O(G4)_{x₄} (IV)
- 50 à 70 % en poids d'un mélange d'alkylpolyglycosides de formules (V) et (VI) :

   R₅O(G₅)_{x₅} (V)

   R₆O(G₆)_{x₆} (VI)

   dans lesquelles R₁, R₂, R₃, R₄, R₅, R₆, G₁, G₂, G₃, G₄, G₅, G₆, x₁, x₂, x₃, x₄, x₅ et x₆ sont tels que définis précédemment.

Les compositions préférées dans le cadre de la présente invention sont les compositions comprenant :
- 10 à 40 % en poids d'un mélange d'alkylpolyglycosides tel que défini précédemment,
- 90 à 60 % en poids d'un ou plusieurs alcools tels que définis précédemment.

Une composition particulièrement préférée dans le cadre de la présente invention est constituée de :
* 19,9 % en poids d'un mélange d'alkylpolyglycosides essentiellement constitué de :
   - 17 % en poids d'un alkylpolyglycoside de formule (I) :

      R₁O(G₁)_{x₁} (I)
   - 19 % en poids d'un alkylpolyglycoside de formule (II) :

      R₂O(G₂)_{x₂} (II°
   - 5 % en poids d'un mélange d'alkylpolyglycosides de formules (III) et (IV):

      R₃O(G₃)_{x₃} (III)

      R₄O(G₄)_{x₄} (IV)
   - 59 % en poids d'un mélange d'alkylpolyglycosides de formules (V) et (VI) :

      R₅O(G₅)_{x₅} (V)

      R₆O(G₆)_{x₆} (VI)

      dans lesquelles R₁, R₂, R₃, R₄, R₅ et R₆ représentent chacun un radical aliphatique, linéaire ou ramifié, ayant respectivement 12, 14, 16, 18, 20 et 22 atomes de carbone, G₁, G₂, G₃, G₄, G₅ et G₆ représentent chacun le reste d'un saccharide, x₁, x₂, x₃, x₄, x₅ et x₆ représentent chacun un nombre compris entre 1 et 5 ;
* 80,1 % en poids d'un ou plusieurs alcools de formule R'OH, dans laquelle R' est un radical aliphatique, linéaire ou ramifié, ayant de 12 à 22 atomes de carbone, et de préférence d'un mélange constitué des alcools dont la partie alkyle est identique à la partie alkyle R₁, R₂, R₃, R₄, R₅ et R₆ des alkylpolyglycosides précités.

Les alkylpolyglycosides de formules (I), (II), (III), (IV), (V) et (VI) précités peuvent comporter, à titre de reste de saccharide représenté respectivement par G₁, G₂, G₃, G₄, G₅ et G₆ un reste de glucose ou dextrose, fructose, galactose, maltose, maltotriose, lactose, cellobiose, mannose, ribose, dextrane, talose, allose, xylose, levoglucosane, cellulose ou amidon.

Avantageusement, G₁, G₂, G₃, G₄, G₅ et G₆ représentent chacun un reste de glucose.

Il est en outre à noter que chaque unité de la partie polyoside de l'alkylpolyglycoside peut être sous forme anomérique α ou β, et le reste de saccharide peut être de type furanoside ou pyranoside.

Les indices x₁, x_{2'} x₃, x₄, x₅ et x₆ représentent le degré de polymérisation moyen du reste de saccharide. De préférence, ces indices représenteront un nombre compris entre 1,05 et 2,5, de préférence encore entre 1,1 et 2.

L'expression "alkylpolyglycoside" utilisée dans le cadre de la présente demande désigne donc indifféremment les alkylmonoosides (degré de polymérisation égal à 1) ou les alkylpolyglycosides (degré de polymérisation supérieur à 1).

Les alkylpolyglycosides de formules (I), (II), (III), (IV), (V) et (VI) sont des composés dont les radicaux alkyles comportent des chaînes de longueur déterminée. Ces composés peuvent toutefois contenir, en outre, des proportions mineures de composés de même nature dont les radicaux alkyles comportent une chaîne plus longue et/ou plus courte, de tels composés provenant notamment des alcools gras généralement d'origine naturelle ou synthétique utilisés comme matière de départ pour la synthèse de ces alkylpolyglycosides.

L'expression "essentiellement constitué" utilisée dans le cadre de la présente demande et des revendications pour caractériser le mélange d'alkylpolyglycosides précité doit donc s'entendre comme n'excluant pas la présence, au sein du mélange d'alkylpolyglycosides, de composés dont les radicaux alkyles ont 10 ou 24 atomes de carbone, en une quantité cumulée maximale de 5 % en poids, et de préférence de 1 % en poids rapportée au poids total du mélange d'alkylpolyglycosides.

Les compositions à base d'alkylpolyglycosides et d'alcools gras conformes à la présente invention peuvent être préparées par simple mélange de leurs constituants en des proportions prédéterminées souhaitées.

A l'échelle industrielle, on les préparera de préférence selon l'une des deux voies classiquement utilisées pour la synthèse des alkylpolyglycosides, et par exemple par réaction, en milieu acide, entre un alcool gras et un saccharide disposant d'un OH anomérique, tel que le glucose ou le dextrose.

De telles voies de synthèse sont bien connues et ont été décrites dans de nombreux documents et en particulier dans les documents de la demanderesse rappelés précédemment.

Le cas échéant, cette synthèse pourra être complétée par des opérations de neutralisation, de filtration, de distillation ou d'extraction partielle de l'alcool gras en excès ou de décoloration.

Les compositions à base alkylpolyglycosides et d'alcools gras conformes à la présente invention peuvent être utilisées, à titre d'émulsionnant principal, pour la préparation d'émulsions fluides variées.

Ainsi, selon un deuxième aspect, la présente demande vise à couvrir des émulsions fluides comprenant au moins une phase aqueuse et une phase huileuse et, à titre d'émulsionnant principal, une composition à base d'alkylpolyglycosides et d'alcools gras telle que définie précédemment.

D'une façon générale, une telle émulsion comprendra de 1 à 25 % en poids, de préférence de 1 à 10 % et de préférence encore 3 % en poids de la composition précitée.

La phase huileuse constitutive de l'émulsion peut être constituée par le ou les alcools gras constitutifs de la composition émulsionnante de l'invention, sans qu'il ne soit nécessaire de mettre en oeuvre une autre huile. Mais plus généralement, on utilisera une huile choisie parmi les huiles suivantes :
- les huiles d'origine végétale, telles que l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula ;
- les huiles végétales modifiées telles que les produits connus sous les dénominations INCI, Apricot Kernel Oil PEG-6 esters et Olive Oil PEG-6 esters ;
- les huiles d'origine naturelle, telles que le perhydrosqualène, le squalène ;
- les huiles minérales, telles que l'huile de paraffine ou huile de vaseline, et les huiles minérales, notamment issues de coupes pétrolières, telles que les isoparaffines, ayant un point d'ébullition compris entre 300 et 400°C ;
- les huiles synthétiques, notamment les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les triglycérides comme le triheptanoate de glycérol, les alkylbenzoates, les isoparaffines, les polyalphaoléfines, les polyoléfines, les isoalcanes de synthèse comme l'isohexadecane, l'isododécane et les huiles de silicone. Parmi ces dernières, on peut plus particulièrement citer les diméthylpolysiloxanes, méthylphénylpolysiloxanes, les silicones modifiés par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiés par des groupements fluorés, des silicones cycliques et des silicones modifiés par des groupements alkyles.

D'une façon générale, les émulsions conformes à la présente invention comprendront jusqu'à 50% et de préférence entre 5 et 30 % en poids de phase huileuse telle que définie précédemment.

Ces émulsions peuvent être préparées par simple dispersion d'une phase grasse constituée de la composition précitée et éventuellement d'une ou plusieurs huiles telles que décrites ci-dessus, dans une phase hydrophile, généralement de l'eau ou un solvant hydrophile.

La dispersion peut être réalisée à chaud ou à froid en fonction du point de fusion de la composition émulsionnante, tous les constituants devant être liquides au moment du mélange.

Les émulsions ainsi obtenues se différencient de celles susceptibles d'être obtenues à partir des compositions émulsionnantes de l'état de la technique, par le fait qu'elles sont stables et fluides, comme il sera démontré plus loin, sans utilisation de co-tensioactif ni de stabilisant.

L'invention sera illustrée plus en détail par les exemples suivants, donnés uniquement à titre illustratif.

### EXEMPLE 1

Procédé de préparation d'une composition à base d'alkylpolyglycosides et d'alcools gras selon l'invention.

On introduit dans un réacteur polyvalent une coupe d'alcools gras constituée en pourcentage pondéral de 16,5 % d'alcool en C₁₂, 18,5 % d'alcool en C₁₄, 4,5 % d'alcools en C₁₆₋₁₈ et 60,5 % d'alcools en C₂₀₋₂₂.

On introduit également dans un réacteur du glucose, de sorte que le rapport molaire entre l'alcool gras et le glucose soit de : 6/1.

On fait ensuite réagir le glucose avec l'alcool gras pendant 5 heures à une température d'environ 100°C en présence d'un catalyseur acide sous vide partiel.

Après neutralisation du catalyseur, la composition obtenue comprend :
- 80,1 % d'alcool gras,
- 3,3 % d'APG en C₁₂,
- 3,7 % d'APG en C₁₄,
- 0,9 % d'APG en C₁₆₋₁₈,
- 12,0 % d'APG en C₂₀₋₂₂.

### EXEMPLES COMPARATIFS 1 à 7

On a préparé sept autres compositions à base d'alkylpolyglycosides et d'alcools gras afin d'étudier notamment l'influence de la nature du mélange d'alkylpolyglycosides sur les propriétés obtenues.

Ces compositions ont été préparées en suivant le protocole expérimental décrit à l'exemple 1, en choisissant la coupe d'alcools gras appropriée.

Les compositions des mélanges d'alkylpolyglycosides et d'alcools gras ainsi obtenues ont été mentionnées dans le tableau I ci-après.

### MISE EN EVIDENCE DES PROPRIETES DES COMPOSITIONS CONFORMES A L'INVENTION

Afin de mettre en évidence les propriétés particulières des compositions à base d'alkylpolyglycosides et d'alcools gras conformes à la présente invention, on a réalisé diverses émulsions au moyen des compositions de l'exemple 1 ainsi que des compositions des exemples de comparaison 1 à 7.

Ces émulsions ont été préparées de la façon suivante :

On porte un mélange constitué d'une composition émulsionnante et d'une phase huileuse à une température supérieure au point de fusion de la composition d'alkylpolyglycosides, de manière à obtenir un mélange liquide.

La phase aqueuse ou un solvant polaire est chauffé à la même température.

Les deux phases (huileuse et aqueuse) sont ensuite homogénéisées au moyen d'un appareil Silverson par exemple pendant une durée de 3 à 6 min à 4 000 rpm.

Les émulsions sont ensuite refroidies jusqu'à température ambiante sous agitation lente type ancre.

Ces émulsions présentent les compositions suivantes :
- compositions émulsionnantes selon l'invention ou selon un exemple comparatif : 3 %,
- phase grasse : 10 %,
- eau : 75 %.

Deux études ont été réalisées en faisant varier la nature de la phase grasse.
Etude 1 : phase grasse = Octanoate de cétéaryle
Etude 2 : phase grasse = Caprique caprylique triglycéride.

La stabilité des émulsions ainsi préparées est contrôlée après vieillissement à 40°C.

La viscosité des émulsions exprimée en cP.s est mesurée après vieillissement à température ambiante à l'aide d'un viscosimètre Brookfield.

Les résultats obtenus ont été reportés au tableau II ci-après :

Dans ce tableau :
M signifie : mois
DPH signifie : déphase
J signifie : jours.

Comme le montre le tableau II, les résultats obtenus démontrent que seule la composition de l'invention représentée par la composition de l'exemple 1 présente simultanément de bonnes propriétés de fluidité et de stabilité.

Les compositions des exemples comparatifs, bien que constituées également par des mélanges d'alkylpolyglycosides et d'alcools gras, ne permettent pas d'obtenir simultanément ces deux propriétés, ce qui démontre l'aspect critique des compositions selon l'invention.

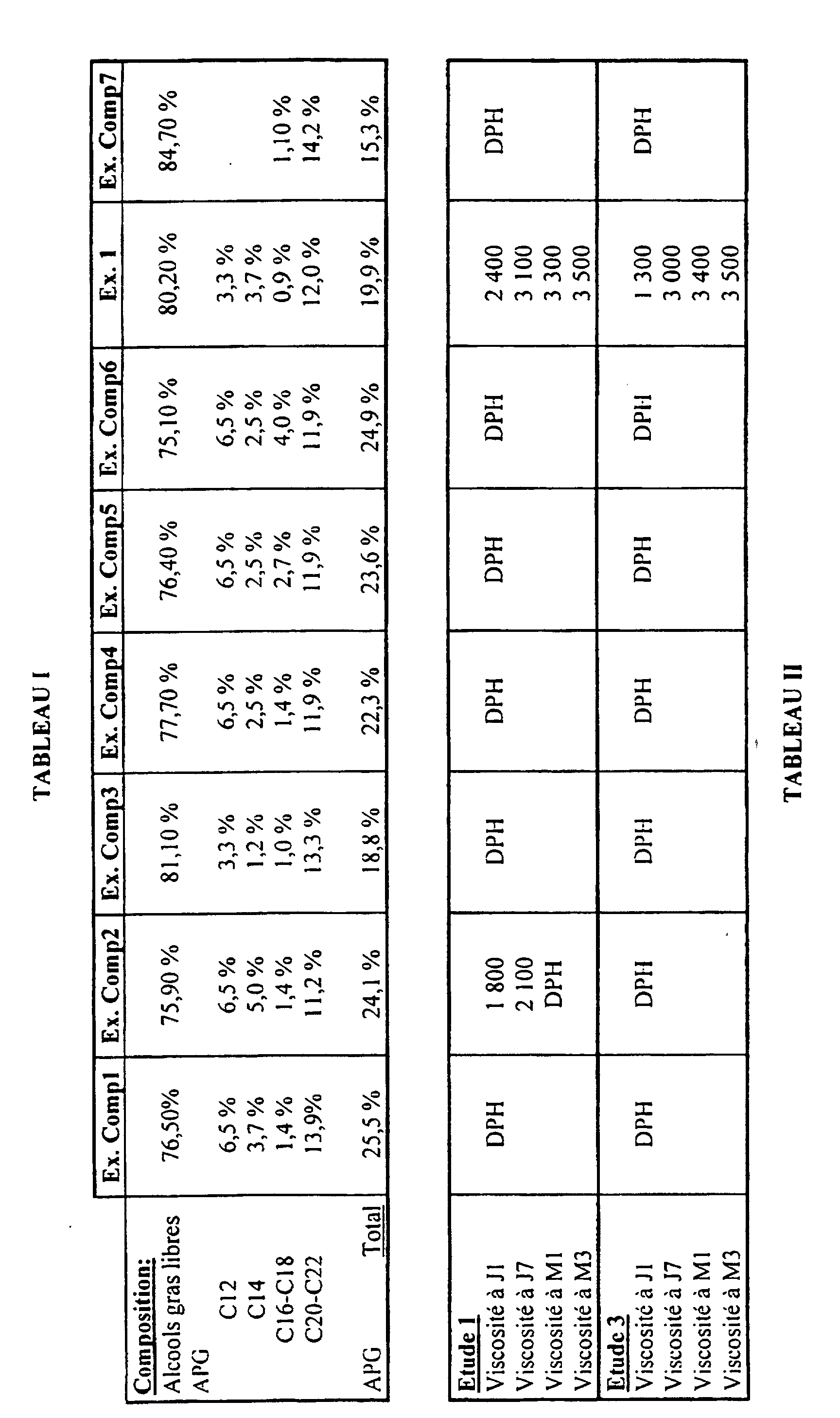

On donnera ci-après plusieurs exemples d'émulsions fluides stables susceptibles d'être préparées par la mise en oeuvre des compositions selon l'invention.

| Lait corporel | |
|---|---|
| Composition selon l'invention | 3 % |
| Alkylbenzoate | 3 % |
| Cyclométhicone | 1 % |
| Diméthicone | 3 % |
| Huile de jojoba | 2 % |
| Eau | qsq 100 % |

| Caractéristiques | |
|---|---|
| Aspect = | lait |
| Viscosité = | 3 000 mPa.S |
| pH = | 5,7 |

| Lait de toilette bébé | |
|---|---|
| Composition selon l'invention | 3 % |
| Huile de paraffine | 2 % |
| Cétéaryloctanoate | 3 % |
| Huile de tournesol | 2 % |
| Huile de bourrache | 3 % |
| eau | qsq 100 % |

| Caractéristiques | |
|---|---|
| Aspect = | lait |
| Viscosité = | 4 000 mPa.S |
| pH = | 6 |

| Lait démaquillant | |
|---|---|
| Composition selon l'invention | 4 % |
| Cétéaryloctanoate | 20 % |
| Glycérine | 5 % |
| Eau | qsq 100 % |

| Caractéristiques | |
|---|---|
| Aspect = | lait |
| Viscosité = | 4 500 mPa.S |
| pH = | 7 |

| Lait apaisant après-soleil | |
|---|---|
| Composition selon l'invention | 2,5 % |
| SEPICALM® V6 | 1,0% |
| Isohexadécanne | 3 % |
| Squalane | 5 % |
| Beurre de karité | 2 % |
| Huile de noisette | 1 % |
| Eau | qsp 100 % |

| Caractéristiques | |
|---|---|
| Aspect = | lait |
| Viscosité = | 5 500 mPa.S |
| pH = | 5,5 |

## Revendications

1. Compositions à base d'alkylpolyglycosides et d'alcools gras, **caractérisées en ce qu'**elles comprennent :
* 5 à 60% en poids d'un mélange d'alkylpolyglycosides essentiellement constitué de :
- 10 à 25 % en poids d'un alkylpolyglycoside de formule (I) :
R₁O(G₁)_{x₁} (I)
- 10 à 30 % en poids d'un alkylpolyglycoside de formule (II) :
R₂O(G2)_{x₂} (II)
- 0 à 10 % en poids d'un mélange d'alkylpolyglycosides de formules (III) et (IV):
R₃O(G₃)_{x₃} (III)
R₄O(G4)_{x₄} (IV)
- 40 à 80 % en poids d'un mélange d'alkylpolyglycosides de formules (V) et (VI) :
R₅O(G₅)_{x₅} (V)
R₆O(G₆)_{x₆} (VI)
dans lesquelles R₁, R₂, R₃, R₄, R₅ et R₆ représentent chacun un radical aliphatique, linéaire ou ramifié, ayant respectivement 12, 14, 16, 18, 20 et 22 atomes de carbone, G₁, G₂, G₃, G₄, G₅ et G₆ représentent chacun le reste d'un saccharide, x₁, x₂, x₃, x₄, x₅ et x₆ représentent chacun un nombre compris entre 1 et 5 ;
* 95 à 40 % en poids d'un ou plusieurs alcools de formule R'OH, dans laquelle R' est un radical aliphatique, linéaire ou ramifié, ayant de 12 à 22 atomes de carbone, et de préférence d'un mélange constitué des alcools dont la partie alkyle est identique à la partie alkyle R₁, R₂, R₃, R₄, R₅ et R₆ des alkylpolyglycosides précités.

2. Compositions selon la revendication 1, **caractérisées en ce que** le mélange d'alkylpolyglycosides précité est essentiellement constitué de :
- 10 à 20 % en poids d'un alkylpolyglycoside de formule (I) :
R₁O(G₁)_{x₁} (I)
- 15 à 25 % en poids d'un alkylpolyglycoside de formule (II) :
R₂O(G₂)_{x₂} (II)
- 0 à 10 % en poids d'un mélange d'alkylpolyglycosides de formules (III) et (IV):
R₃O(G₃)_{x₃} (III)
R₄O(G₄)_{x₄} (IV)
- 50 à 70 % en poids d'un mélange d'alkylpolyglycosides de formules (V) et (VI) :
R₅O(G₅)_{x₅} (V)
R₆O(G₆)_{x₆} (VI)
dans lesquelles R₁, R₂, R₃, R₄, R₅, R₆, G₁, G₂, G₃, G₄, G₅, G₆, x₁, x₂, x₃, x₄, x₅ et x₆ sont tels que définis à la revendication 1.

3. Compositions selon la revendication 1 ou 2, **caractérisées en ce qu'**elles comprennent :
- 10 à 40 % en poids d'un mélange d'alkylpolyglycosides tel que défini à la revendication 1 ou 2,
- 90 à 60 % en poids d'un ou plusieurs alcools tels que définis à la revendication 1 ou 2.

4. Compositions selon l'une des revendications 1 à 3, **caractérisées en ce qu'**elles comprennent :
* 19,9 % en poids d'un mélange d'alkylpolyglycosides essentiellement constitué de :
- 17 % en poids d'un alkylpolyglycoside de formule (I) :
R₁O(G₁)_{x₁} (I)
- 19 % en poids d'un alkylpolyglycoside de formule (II) :
R₂O(G₂)_{x₂} (II)
- 5 % en poids d'un mélange d'alkylpolyglycosides de formules (III) et (IV):
R₃O(G3)_{x₃} (III)
R₄O(G₄)_{x₄} (IV)
- 59 % en poids d'un mélange d'alkylpolyglycosides de formules (V) et (VI) :
R₅O(G₅)_{x₅} (V)
R₆O(G₆)_{x₆} (VI)
dans lesquelles R₁, R₂, R₃, R₄, R₅ et R₆ représentent chacun un radical aliphatique, linéaire ou ramifié, ayant respectivement 12, 14, 16, 18, 20 et 22 atomes de carbone, G₁, G₂, G₃, G₄, G₅ et G₆ représentent chacun le reste d'un saccharide, x_{1'} x₂, x₃, x₄, x₅ et x₆ représentent chacun un nombre compris entre 1 et 5 ;
* 80,1 % en poids d'un ou plusieurs alcools de formule R'OH, dans laquelle R' est un radical aliphatique, linéaire ou ramifié, ayant de 12 à 22 atomes de carbone, et de préférence d'un mélange constitué des alcools dont la partie alkyle est identique à la partie alkyle R₁, R₂, R₃, R₄, R₅ et R₆ des alkylpolyglycosides précités.

5. Emulsions fluides et stables comprenant au moins une phase aqueuse et une phase huileuse et, à titre d'émulsionnant principal, une composition à base d'alkylpolyglycosides et d'alcools gras tels que définis selon l'une quelconque des revendications 1 à 4.

6. Emulsions selon la revendication 5, **caractérisées en ce qu'**elles comprennent de 1 à 25 % en poids, de préférence de 1 à 10 % en poids de la composition émulsionnante précitée et jusqu'à 50 % en poids de la phase huileuse précitée.

## Claims

1. Compositions based on alkyl polyglycosides and fatty alcohols, **characterised in that** they comprise :
* 5 to 60 % by weight of a mixture of alkyl polyglycosides essentially consisting of :
- 10 to 25 % by weight of an alkyl polyglycoside of formula (I) :
R₁O(G₁)_{x₁} (I)
- 10 to 30 % by weight of an alkyl polyglycoside of formula (II) :
R₂O(G₂)_{x₂} (II)
- 0 to 10 % by weight of a mixture of alkyl polyglycosides ot formulae (III) and (IV):
R₃O(G₃)_{x₃} (III)
R₄O(G₄)_{x₄} (IV)
- 40 to 80 % by weight of a mixture of alkyl polyglycosides of formulae (V) and (VI) :
R₅O(G₅)_{x₅} (V)
R₆O(G₆)_{x₆} (VI)
in which R₁, R₂, R₃, R₄, R₅ and R₆ each represent a linear or branched aliphatic radical having 12, 14, 16, 18, 20 and 22 carbon atoms respectively, G₁, G₂, G₃, G₄, G₅ and G₆ each represent a saccharide residue, x₁, x₂, x₃, x₄, x₅ and x₆ each represent a number between 1 and 5 ;
* 95 to 40 % by weight of one or more alcohols of formula R'OH, in which R' is a linear or branched aliphatic radical having 12 to 22 carbon atoms, and preferably of a mixture consisting of alcohols the alkyl part of which is identical to the alkyl part R₁, R₂, R₃, R₄, R₅ and R₆ of the alkyl polyglycosides mentioned above.

2. Compositions according to claim 1, **characterised in that** the mixture of alkyl polyglycosides mentioned above essentially consists of :
- 10 to 20 % by weight of an alkyl polyglycoside of formula (I) :
R₁O(G₁)_{x₁} (I)
- 15 to 25 % by weight of an alkyl polyglycoside of formula (II) :
R₂O(G₂)_{x₂} (II)
- 0 to 10 % by weight of a mixture of alkyl polyglycosides of formulae (III) and (IV):
R₃O(G₃)_{x₃} (III)
R₄O(G₄)_{x₄} (IV)
- 50 to 70 % by weight of a mixture of alkyl polyglycosides of formulae (V) and (VI) :
R₅O(G₅)_{x₅} (V)
R₆O(G₆)_{x₆} (VI)
in which R₁, R₂, R₃, R₄, R₅, R₆, G₁, G₂, G₃, G₄, G₅, G₆, x₁, X₂, X₃, X₄, x₅ and x₆ are as defined in claim 1.

3. Compositions according to claim 1 or 2, **characterised in that** they comprise :
- 10 to 40 % by weight of a mixture of alkyl polyglycosides as defined in claim 1 or 2,
- 90 to 60 % by weight of one or more alcohols as defined in claim 1 or 2.

4. Compositions according to one of claims 1 to 3, **characterised in that** they comprise :
* 19.9 % by weight of a mixture of alkyl polyglycosides essentially consisting of :
- 17 % by weight of an alkyl polyglycoside of formula (I) :
R₁O(G₁)_{x₁} (I)
- 19 % by weight of an alkyl polyglycoside of formula (II) :
R₂O(G₂)_{x₂} (II)
- 5 % by weight of a mixture of alkyl polyglycosides of formulae (III) and (IV):
R₃O(G₃)_{x₃} (III)
R₄O(G₄)_{x₄} (IV)
- 59 % by weight of a mixture of alkyl polyglycosides of formulae (V) and (VI) :
R₅O(G₅)_{x₅} (V)
R₆O(G₆)_{x₆} (VI)
in which R₁, R₂, R₃, R₄, R₅ and R₆ each represent a linear or branched aliphatic radical having 12, 14, 16, 18, 20 and 22 carbon atoms respectively, G₁, G₂, G₃, G₄, G₅ and G₆ each represent a saccharide residue, x₁, x₂, x₃, x₄, x₅ and x₆ each represent a number between 1 and 5 ;
* 80.1 % by weight of one or more alcohols of formula R'OH, in which R' is a linear or branched aliphatic radical having 12 to 22 carbon atoms, and preferably of a mixture consisting of alcohols the alkyl part of which is identical to the alkyl part R₁, R₂, R₃, R₄, R₅ and R₆ of the alkyl polyglycosides mentioned above.

5. Stable, fluid emulsions comprising at least an aqueous phase and an oily phase and, as main emulsifier, a composition based on alkyl polyglycosides and fatty alcohols as defined in any one of claims 1 to 4.

6. Emulsions according to claim 5, **characterised in that** they comprise 1 to 25 % by weight, preferably 1 to 10 % by weight of the emulsifying composition mentioned above and up to 50 % by weight of the oily phase mentioned above.

## Patentansprüche

1. Zusammensetzungen auf der Basis von Alkylpolyglykosiden und Fettalkoholen, **dadurch gekennzeichnet, dass** sie umfassen:
* 5 bis 60 Gew.-% eines Gemischs von Alkylpolyglykosiden, im Wesentlichen bestehend aus:
- 10 bis 25 Gew.-% eines Alkylpolyglykosids der Formel (I):
R₁O(G₁)_{x₁} (I)
- 10 bis 30 Gew.-% eines Alkylpolyglykosids der Formel (II):
R₂O(G₂)_{x₂} (II)
- 0 bis 10 Gew.-% Gemischs von Alkylpolyglykosiden der Formeln (III) und (IV):
R₃O(G₃)_{x₃} (III)
R₄O(G₄)_{x₄} (IV)
- 40 bis 80 Gew.-% eines Gemischs von Alkylpolyglykosiden der Formeln (V) und (VI):
R₅O(G₅)_{x₅} (V)
R₆O(G₆)_{x₆} (VI)
wobei R₁, R₂, R₃, R₄, R₅ und R₆ jeweils einen geradkettigen oder verzweigten aliphatischen Rest mit 12, 14, 16, 18, 20 bzw. 22 Kohlenstoffatomen darstellen, G₁, G₂, G₃, G₄, G₅ und G₆ jeweils einen Rest eines Saccharids darstellen, x₁, x₂, x₃, x₄, x₅ und x₆ jeweils eine Zahl zwischen 1 und 5 darstellen;
* 95 bis 40 Gew.-% eines oder mehrerer Alkohole der Formel R'OH, wobei R' einen geradkettigen oder verzweigten aliphatischen Rest mit 12 bis 22 Kohlenstoffatomen darstellt, und vorzugsweise eines Gemischs, bestehend aus Alkoholen, deren Alkylteil identisch mit dem Alkylteil R₁, R₂, R₃, R₄, R₅ und R₆ des vorgenannten Alkylpolyglykosids ist.

2. Zusammensetzungen gemäß Anspruch 1, **dadurch gekennzeichnet, dass** das vorgenannte Gemisch von Alkylpolyglykosiden im Wesentlichen aus:
- 10 bis 20 Gew.-% eines Alkylpolyglykosids der Formel (I):
R₁O(G₁)_{x₁} (I)
- 15 bis 25 Gew.-% eines Alkylpolyglykosids der Formel (II):
R₂O(G₂)_{x₂} (II)
- 0 bis 10 Gew.-% eines Gemischs von Alkylpolyglykosiden der Formeln (III) und (IV):
R₃O(G₃)_{x₃} (III)
R₄O(G₄)_{x₄} (IV)
- 50 bis 70 Gew.-% eines Gemischs von Alkylpolyglykosiden der Formeln (V) und (VI):
R₅O(G₅)_{x₅} (V)
R₆O(G₆)_{x₆} (VI)
besteht,
wobei R₁, R₂, R₃, R₄, R₅, R₆, G₁, G₂, G₃, G₄, G₅, G₆, x₁, x₂, x₃, x₄, x₅ und x₆ wie in Anspruch 1 definiert sind.

3. Zusammensetzungen gemäß Anspruch 1 oder 2, **dadurch gekennzeichnet, dass** sie umfassen:
- 10 bis 40 Gew.-% eines Gemischs von Alkylpolyglykosiden, wie in Anspruch 1 oder 2 definiert,
- 90 bis 60 Gew.-% eines oder mehrerer Alkohole, wie in Anspruch 1 oder 2 definiert.

4. Zusammensetzungen gemäß einem der Ansprüche 1 bis 3, **dadurch gekennzeichnet, dass** sie umfassen:
* 19,9 Gew.-% eines Gemischs von Alkylpolyglykosiden, im Wesentlichen bestehend aus:
- 17 Gew.-% eines Alkylpolyglykosids der Formel (I):
R₁O(G₁)_{x₁} (I)
- 19 Gew.-% eines Alkylpolyglykosids der Formel (II):
R₂O(G₂)_{x₂} (II)
- 5 Gew.-% eines Gemischs von Alkylpolyglykosiden der Formeln (III) und (IV):
R₃O(G₃)_{x₃} (III)
R₄O(G₄)_{x₄} (IV)
- 59 Gew.-% eines Gemischs von Alkylpolyglykosiden der Formeln (V) und (VI):
R₅O(G₅)_{x₅} (V)
R₆O(G₆)_{x₆} (VI)
wobei R₁, R₂, R₃, R₄, R₅ und R₆ jeweils einen geradkettigen oder verzweigten aliphatischen Rest mit 12, 14, 16, 18, 20 bzw. 22 Kohlenstoffatomen darstellen, G₁, G₂, G₃, G₄, G₅ und G₆ jeweils einen Rest eines Saccharids darstellen, x₁, x₂, x₃, x₄, x₅ und x₆ jeweils eine Zahl zwischen 1 und 5 darstellen;
* 80,1 Gew.-% eines oder mehrerer Alkohole der Formel R'OH, wobei R' einen linearen oder verzweigten aliphatischen Rest mit 12 bis 22 Kohlenstoffatomen darstellt, und vorzugsweise eines Gemisches, bestehend aus Alkoholen, deren Alkylteil identisch mit dem Alkylteil R₁, R₂, R₃, R₄, R₅ und R₆ des vorgenannten Alkylpolyglykosids ist.

5. Flüssige und stabile Emulsionen, umfassend mindestens eine wässrige Phase und eine ölige Phase und, als Hauptemulgator, eine Zusammensetzung auf der Basis von Alkylpolyglykosiden und Fettalkoholen, wie gemäß einem der Ansprüche 1 bis 4 definiert.

6. Emulsionen gemäß Anspruch 5, **dadurch gekennzeichnet, dass** sie 1 bis 25 Gew.-%, vorzugsweise 1 bis 10 Gew.-% der vorgenannten Emulgatorzusammensetzung und bis zu 50 Gew.-% der vorgenannten öligen Phase umfassen.
